# EUROPEAN PATENT SPECIFICATION

(11) **EP 2 277 546 B1**
(45) Date of publication and mention of the grant of the patent: **15.07.2015**
(21) Application number: 09009569.6
(22) Date of filing: 23.07.2009
(51) Int. Cl.: A61K 9/08, A61K 47/18, A61K 47/20, A61K 47/40, A61K 47/48, A61K 31/167

(54) **Stable ready to use injectable paracetamol formulation**
Stabile, gebrauchsfertige, injizierbare Paracetamolformulierung
Formule de paracétamol injectable, stable et prête à utiliser

(43) Date of publication of application: 26.01.2011
(73) Proprietor: Uni-Pharma Kleon Tsetis Pharmaceutical Laboratories S.A., 145 64 Kifissia (GR); Tseti, Ioulia, 145 61 Kifissia (GR)
(72) Inventor: Tseti, Ioulia, 145 61 Kifissia (GR)
(74) Representative: Wibbelmann, Jobst

(56) References cited:
- WO-A1-03/051398
- WO-A1-2009/064928
- WO-A2-2008/021896
- US-A- 4 727 064
- US-A- 6 028 222
- US-A1- 2005 215 520

## Description

The present invention refers to a pharmaceutical composition comprising Paracetamol for parenteral administration by IV infusion, with an optimum pH 6.0 (ranging between 5.5 and 6.5) comprising at least one stabilizing and one dissolving substance of paracetamol in solution such as a cyclodextrin, EDTA, monothioglycerol (MTG), in a suitable concentration, able to stabilize and solubilize the paracetamol.

### Description

### Field of the Invention

The present invention relates to an injectable liquid paracetamol composition.

### Background of the Invention

Paracetamol is considered to be the main active metabolite of phenacetin and acetanidile having analgesic and antipyretic properties. Paracetamol has equivalent analgesic and antipyretic action to that of aspirin whilst it expresses weak anti-inflammatory action therefore its use in inflammatory rheumatic diseases is limited.

A large number of pharmaceutical preparations to be administered orally or even topically are known. However, it is difficult to obtain a pharmaceutical preparation for injection and particularly, a ready-to-use solution for intravenous perfusion, due to the fact that paracetamol is not very soluble in water and its solutions in aqueous medium are unstable in the presence of oxygen and/or light, being decomposed through a plurality of degradation pathways which are well known and are described for example in the article "Stability of aqueous solutions of N-acetyl-p-aminophenol", by K.T. Koshy and J.L. Lach, J. Pharmaceutical Sciences, Vol 50 (2) (February 1961), p. 113-118. This instability in aqueous medium is shown by the appearance of degradation substances causing a coloring in the solution. The different substances causing the coloring of the solution include benzoquinoimines which are hepatotoxic in humans.

However, the development of color in pharmaceutical solutions and especially in injectable formulations, which must be completely transparent, involves a serious problem, because the presence of said color is indicative of the existence of unwanted compounds in the formulation and therefore leads to the rejection of the injectable product without being used.

One of the causes of paracetamol degradation is based on chemical oxidation reactions in which the oxygen present in the solution is the main precursor of this degradation. The secondary cause of degradation may be the deacetylation of the amino group generating p-aminophenol which is also quickly degraded producing p-benzoquinoneimine. This deacetylation takes places both at acid pH and (much faster) at basic pH once the phenolate form is present.

Obtaining stable paracetamol solutions in aqueous medium can be solved by means of several joint actions.
1) Establishing an optimal pH in which the formation of 4-aminophenol is prevented or minimized, as has been indicated by K. Thomas Koshy and Jon L. Lach in the previous indicated reference "Stability of aqueous solutions of N-acetyl-p-aminophenol", J. of Phar. Sci., Vol 50 No. 2 (1961), 113-118, the hydrolysis of the acetate group of paracetamol is minimized between pH= 4.5 and pH 6.0.
2) Preventing the presence of oxygen in solution. This action is described in Spanish patent no. 2,201,316, from the validation in Spain of European patent EP 858,329 B1, issued to Pharmatop SCR. This document discloses a process whereby paracetamol oxidation is prevented by means of eliminating the main element activating the reaction, oxygen, with nitrogen bubbling. By further keeping the solution in a completely hermetic bottle, the stability of paracetamol in solution is ensured for long time periods, with minimal impurity levels and the total absence of color in the solution. It may be presumed that this product of the prior art must be kept in suitable bottles preventing the incorporation of oxygen into the solution and therefore these solutions cannot be stored in individual oxygen-permeable bottles such as plastic materials.

WO 2009/064928 A1 discloses aqueous solutions for intravenous administration comprising acetaminophen (paracetamol), and at least one antioxidant (such as alpha-thioglycerol (monothioglycerol)). In some embodiments, the intravenous aqueous solutions further comprise EDTA.

The present invention concerns the stable aqueous paracetamol solution for use in IV infusion of claim 1 with an optimum pH. An optimum pH is typically a pH of 6.0, ranging between 5.5 and 6.5. A preferred pharmaceutical composition according to the invention comprises:

| **Ingredient** | **Quantity/100 ml** |
|---|---|
| Paracetamol | 1000 mg |
| Hydroxypropyl-beta-cyclodextrin | 666 mg |
| Monothioglycerol | 10 mg |
| EDTA | 10 mg |
| NaCl | 600 mg |
| Disodium phosphate dihydrate | 35.6 mg |
| Water for Injection to | 100 ml |
| Final pH (HCl or NaOH) 1M | 5.5-6.5 |

Typically, the 2-hydroxypropyl-beta-cyclodextrin is provided in a concentration between 0.2% m/v and 20% m/v. Preferably the 2-hydroxypropyl-beta-cyclodextrin is present in a concentration between 0.2% m/v and 6.0% m/v, more preferably between 0.5% and 3.0% m/v.

The stable aqueous paracetamol solution according to the invention may have a pH between 4.0 and 7.0. Typically, the solution may be a buffer with a buffer composition selected from at least one of the acid form and the ionized form of: citric, malic, acetic, sorbic, phosphoric, fumaric, lactic, gluconic and tartaric acids or mixtures thereof. Preferably, the pH is between 5.5 and 6.5 and more preferably the pH is adjusted to 6. A typical buffer includes phosphate or sodium citrate/acetate.

The stable aqueous paracetamol solution according to the invention may further comprise isotonizing agents, preferably sodium chloride

The stable aqueous paracetamol solution for IV infusion may be sterilized by heat or by filtration.

A typical concentration of paracetamol is between 0.20% and 10% m/v, preferably 0.5% and 1.5% m/v.

The aqueous medium of the solution according to the invention may have been deoxygenated by a water-insoluble inert gas (N₂).

The compositions according to the invention will be administered intravenously and they are stable when stored for more than 24 months at room temperature. Moreover, the compositions may be even stable when stored for more than 3 months at elevated temperatures.

A composition may be prepared in solution and stored in clear glass containers or bottles made of a polymer material such as polyethylene, or in soft material bags made from polyethylene, polyvinylchloride or polypropylene.

According to the invention, a stable aqueous solution is provided, comprising Paracetamol and 2-hydroxypropyl-beta-cyclodextrin with EDTA, and monothioglycerol, in a suitable concentrations.

The molar ratio of Paracetamol to 2-hydroxypropyl beta-cyclodextrin is 100:1 to 0.1:1 , most preferably 5:1.

Typically, the solution comprises 2 mg to 200 mg, preferably more than 5 mg, most preferably 10 mg, Paracetamol per millilitre solution.

The EDTA comprises 0.0015 to 1 mg, most preferably 0.1 mg, per millilitre solution.

The monothioglycerol comprises 0.015 to 1 mg, most preferably 0.1 mg, per millilitre solution.

Advantageously, the solution is in the form of a unit dose that does not exceed 100 millilitres.

The inventor has found a way to prepare a stable aqueous solution comprising Paracetamol and 2-hydroxypropyl-beta-cyclodextrin, which is not only capable of having a concentration of Paracetamol of more than 10 mg per millilitre of solution, but is also stable and does not need to be refrigerated when packed in clear glass sealed vials, or in stoppered glass vials or in bottles made of a polymer material such as polyethylene, or in soft material bags made from polyethylene, polyvinyl chloride or polypropylene.

By "stable" is meant that the solution can be stored for at least 24 months at room temperature and at least 3 months at elevated temperature (40°C) without the appearance of colour and particulate matter which is visible to the eye.

The use of monothioglycerol (MTG) and EDTA has been found to not only increase the Paracetamol solubility to the extent that it is possible to dissolve 1000 mg of Paracetamol into a final volume of 100 ml but also effectively stabilises the solution preventing the formation of particulate matter and colour at elevated temperature in ampoules, vials and bags.

The solution may be formulated in unit dose form, each unit dose containing from 100 mg to 1500 mg Paracetamol inclusive, more preferably from 600 mg to 1000 mg inclusive, most preferably 1000 mg, in a volume not exceeding 100 millilitres.

The 2-hydroxypropyl beta-cyclodextrin (HPBCD) is selected from derivatives with a degree of substitution of between 2.5 and 10 hydroxypropyl substituents per beta-cyclodextrin molecule, more preferably between 3.5 and 8 hydroxypropyl substitutents per beta-cyclodextrin molecule. The molar ratio of Paracetamol to 2-hydroxypropyl beta-cyclodextrin is 100:1 to 0.1:1 , more preferably 10:1, most preferably 5:1.

The injectable stabilised solution of the invention may be prepared by methods known in the art

The stabilised injectable solution of the invention may be packed into suitable containers known in the art (for example glass ampoules, vials, cartridges, glass sealed vials, or in stoppered glass vials or in bottles made of a polymer material such as polyethylene, or in soft material bags made from polyethylene, polyvinyl chloride or polypropylene). The glass should preferably be clear glass.

The stabilized injectable solution of the invention is suitable for intravenous use.

The stabilized injectable solution of the invention need not be stored under refrigerated conditions to provide a shelf life of at least 24 months, saving refrigeration costs during transport and storage, and alleviating patient discomfort during administration.

The antioxidants of the invention show advantages over a control solution containing no antioxidant and solutions containing other antioxidants, namely NAC. Tables 3-6 below show stability evaluations of 1000 mg per 100 ml Paracetamol formulations prepared according to the process which is described in EXAMPLE 4 and stored at 40°C for 3 and 6 months respectively. It is evident from Tables 3-6 that all formulations, according to the invention, are stable after 3 months at 40°C and therefore possible formulations, except NAC which is not stable. The example which contains monothioglycerol and EDTA is the example concerning the present invention.

The invention will now be described in more detail with reference to the following non-limiting examples.

### EXAMPLE 1

The unit composition of a first formulation is provided in Table 1 below:

**Table 1**

| **Ingredient** | **Quantity/100 ml** |
|---|---|
| Paracetamol | 1000 mg |
| Hydroxypropyl-β-cyclodextrin | 666 mg |
| Disodium edetate (EDTA) | 10 mg |
| Monothioglycerol | 10 mg |
| NaCl | 600 mg |
| Disodium phosphate dihydrate | 35.6 mg |
| Water for Injection to | 100 ml |
| Final pH ( HCl or NaOH ) 1M | 5.5-6.5 |

### EXAMPLE 2 (not according to the invention)

The unit composition of a second formulation (Control , No additives) is provided in Table 2 below:

**Table 2**

| **Ingredient** | **Quantity/100 ml** |
|---|---|
| Paracetamol | 1000 mg |
| Hydroxypropyl-β-cyclodextrin | 666 mg |
| NaCl | 600 mg |
| Disodium phosphate dihydrate | 35.6 mg |
| Water for Injection to | 100 ml |
| Final pH ( HCl or NaOH ) 1M | 5.5-6.5 |

### EXAMPLE 3

Laboratory-scale formulations given in Examples 1 and 2 of the present invention were manufactured and filled into clear glass vials and polymer material (soft material bags) and placed on a stability program. Tables 3 - 6 below summarizes the results obtained:

**Table 3**

| **Stability of 1000mg/100ml (Paracetamol- HPBCD) Batches at 40°C For 3 Months** | | | | |
|---|---|---|---|---|
| **Antioxidant** | **Chemical stability** | **pH** | **Appearance** | **Comments** |
| Control (No additives) | Acceptable level of known degradant | downward | Discolouration; physical instability | Not stable (continued to 6 months as control) |
| EDTA 10 mg | Acceptable level of known degradant | slightly downward | Stable | Complies, trial continued |
| NAC 10 mg + EDTA 5 mg | Acceptable level of known degradant | downward | Discolouration; physical instability | Not stable, trial continued |
| Monothioglycerol 20 mg | Acceptable level of known degradant | Stable | Stable | Complies, trial continued |
| Monothioglycerol 10 mg + EDTA 10 mg | Acceptable level of known degradant | Stable | Stable | Complies, trial continued |

**Table 4**

| Stability of 1000mg/100ml (Paracetamol- HPBCD) Batches at 40°C For 6 Months | | | | |
|---|---|---|---|---|
| **Antioxidant** | **Chemical stability** | **pH** | **Appearance** | **Comments** |
| Control (No additives) | Acceptable level of known degradant | Slightly downward | Discolouration; physical instability | Not stable (Control) |
| EDTA 10 mg | Acceptable level of known degradant | Slightly downward | Clear, straw colour Free from visible Particulate matter | Formulation possible |
| NAC 10 mg + EDTA 5 mg | Acceptable level of known degradant | downward | Discolouration; physical instability | Not stable |
| Monothioglycerol 20 mg | Acceptable level of known degradant | Slightly downward | Clear, straw colour Free from visible Particulate matter | Formulation possible |
| Monothioglycerol 10 mg + EDTA 10 mg | Acceptable level of known degradant | Stable | Clear, straw colour Free from visible Particulate matter | Formulation possible |

**Table 5**

| **STABILITY DATA FOR 1000mg/100 ml Paracetamol-_HPB SOLUTIONS (T= 6 months)** | | | | | |
|---|---|---|---|---|---|
| soft material bags Clear glass vials | | | | | |
| **Stabilised by : Control solutions** | | | **Stabilised by: 10 mg EDTA & 10 mg Monothioglycerol** | | |
| | **25°C** | **40°C** | | **25°C** | **40°C** |
| **Appearance** | Clearcolourless solution | Discolouration; physical instability | **Appearance** | Clear colourless solution | Clear, straw coloured solution |
| **pH** | 6 | 5.7 | **pH** | 6 | 5.9 |
| **Particulate Matter** | Free from visible Particulate matter | Free from visible Particulate matter | **Particulate Matter** | Free from visible particulate matter | Free from visible Particulate matter |
| **Assay (HPLC) (% of T=0)** | 100.1 | 98 | **Assay (HPLC) (% of T=0)** | 100 | 99.5 |
| **p-aminophenol%** | <0.005 m/m | 0.5 | **p-aminophenol%** | <0.005 %m/m | 0.02 |
| **Other degradants** | None detected | None detected | **Other degradants** | None detected | None detected |

**Table 6**

| **STABILITY DATA FOR 1000mg/100 ml Paracetamol-_HPB SOLUTIONS (T= 6 months)** | | | | | |
|---|---|---|---|---|---|
| soft material bags | | | | | |
| **Stabilised by: Control solutions** | | | **Stabilised by: 10 mg EDTA & 10 mg Monothioglycerol** | | |
| | **25°C** | **40°C** | | **25°C** | **40°C** |
| **Appearance** | Clear colourless solution | Discolouration; physical instability | **Appearance** | Clear colourless solution | Clear, straw coloured solution |
| **pH** | 6 | 5.4 | **pH** | 6 | 5.8 |
| **Particulate Matter** | Free from visible Particulate matter | Free from visible Particulate matter | **Particulate Matter** | Free from visible particulate matter | Free from visible Particulate matter |
| **Assay (HPLC) (% of T=0)** | 99.7 | 97.5 | **Assay (HPLC) (% of T=0)** | 100 | 99.3 |
| **p-aminophenol** % | <0.005 m/m | 0.8 | **p-aminophenol %** | <0.005 %m/m | 0.03 |
| **Other degradants** | None detected | None detected | **Other degradants** | None detected | None detected |

Control solutions at 40°C for 6 months showed coloured material.

After 24 months at 25°C the solution containing 10 mg EDTA & 10 mg Monothioglycerol remained clear and colourless (both in Clear glass vials and soft material bags), free from visible particulate matter. The associated solution containing NAC 10 mg + EDTA 5 mg was clear but more coloured than the monothioglycerol /EDTA solution.

### EXAMPLE 4

To produce 100 1000mg/100 ml paracetamol units for IV injection, 8000 ml water for injection (WFI) is purged with nitrogen gas to reduce the oxygen. The water was heated to 50°C. Processing continues under a nitrogen gas blanket. 66.675g of HPBCD (DS 4.69) is added to 60 % of the WFI batch volume and is mixed until dissolved. The solution is then allowed to cool to room temperature. The solution is pre-filtered with a 0.45Pg filter, followed by the addition of 1 g MTG, 1 g EDTA, 60 g NaCl and 3.56 g Disodium phosphate dihydrate. The solution is stirred until all the MTG, EDTA, NaCl and Disodium phosphate dihydrate is dissolved. The pH is then adjusted to 6 with HCl 1M. 100 g paracetamol is added to the solution and stirred until dissolved. pH is adjusted to 6, should it be required and made up to 100% volume with WFI. The resultant 1000mg/100 ml paracetamol solution is sterilized by filtration with 0.22Pm filters and filled into pre-sterilized vials or bags, under aseptic conditions. The vials or bags are sealed aseptically under nitrogen. The formulation contains 1000 mg/100 ml paracetamol, as determined by validated HPLC.

## Claims

1. A stable aqueous paracetamol solution for use in IV infusion comprising a 2-hydroxypropyl-beta-cyclodextrin as a stabilizing-dissolving compound wherein the molar ratio of paracetamol to 2-hydroxypropyl-beta-cyclodextrin is 100:1 to 0.1:1, and the aqueous solution comprises 2 mg to 200 mg of paracetamol per ml solution, further comprising EDTA and monothioglycerol as stabilizing compounds, wherein the concentration of EDTA is 0.0015 mg to 1 mg per ml solution and the concentration of monothioglycerol is 0.015 mg to 1 mg per ml solution.

## Patentansprüche

1. Stabile wässrige Paracetamol-Lösung zur Verwendung in IV-Infusion umfassend ein 2-Hydroxypropylbetacyclodextrin als eine stabilisierend-auflösende Verbindung wobei das molare Verhältnis von Paracetamol zu 2-Hydroxypropylbetacyclodextrin 100:1 bis 0,1:1 beträgt, und die wässrige Lösung 2 mg bis 200 mg Paracetamol pro ml Lösung umfasst, des Weiteren umfassend EDTA und Monothioglycerin als stabilisierende Verbindungen, wobei die Konzentration von EDTA 0,0015 mg bis 1 mg pro ml Lösung beträgt und die Konzentration von Monothioglycerin 0,015 mg bis 1 mg pro ml Lösung beträgt.

## Revendications

1. Solution aqueuse stable de paracétamol destinée à une utilisation dans une perfusion IV comprenant une 2-hydroxypropyl-bêta-cyclodextrine comme un composé de stabilisation-dissolution dans laquelle le rapport molaire de paracétamol sur la 2-hydroxypropyl-bêta-cyclodextrine est 100:1 à 0,1:1, et la solution aqueuse comprend 2 mg à 200 mg de paracétamol par ml de solution, comprenant en outre de l'EDTA et du monothioglycérol comme composés de stabilisation, dans laquelle la concentration d'EDTA est 0,0015 mg à 1 mg par ml de solution et la concentration de monothioglycérol est 0,015 mg à 1 mg par ml de solution.
